# EUROPÄISCHE PATENTSCHRIFT

(11) **EP 3 001 817 B1**
(45) Veröffentlichungstag und Bekanntmachung des Hinweises auf die Patenterteilung: **21.09.2016**
(21) Anmeldenummer: 15745453.9
(22) Anmeldetag: 30.07.2015
(51) Int. Cl.: A61L 31/14, A61B 17/064, A61B 17/122

(54) **TEILRESORBIERBARER GEWEBECLIP**
PARTLY RESORBABLE TISSUE CLIP
CLIP DE TISSU PARTIELLEMENT RÉSORBABLE

(30) Priorität: 04.08.2014 DE 102014111038
(43) Veröffentlichungstag der Anmeldung: 06.04.2016
(73) Patentinhaber: Ovesco Endoscopy AG, 72074 Tübingen (DE)
(72) Erfinder: SCHURR, Marc, Prof. Dr., 72072 Tübingen (DE); ANHÖCK, Gunnar, 72764 Reutlingen (DE)
(74) Vertreter: Winter, Brandl, Fürniss, Hübner, Röss, Kaiser, Polte - Partnerschaft mbB
(86) Internationale Anmeldenummer: PCT/EP2015/067496
(87) Internationale Veröffentlichungsnummer: WO 2016/020259

(56) Entgegenhaltungen:
- EP-A1- 2 449 983
- DE-U1- 29 923 545

## Beschreibung

Die vorliegende Erfindung betrifft einen teilresorbierbaren Gewebeclip zum Zusammenheften von Gewebe eines menschlichen oder tierischen Körpers, welcher so beschaffen ist, dass durch Resorption eines resorbierbaren Anteils des Gewebeclips die Klemmwirkung des Gewebeclips mit der Zeit fragmentierungsfrei (d. h. ohne Zerfall in einzelne Metallbruchstücke) nachlässt, bis sich der Gewebeclip selbsttätig von dem Gewebe ablöst und, beispielsweise bei chirurgischen, gastroenterologischen oder proktologischen Eingriffen, ohne weitere operative Intervention von dem Körper ausgeschieden werden kann.

### Hintergrund der Erfindung

In der modernen Endoskopie oder Chirurgie kommen Gewebeclips beispielsweise zum Abklemmen von Gewebeabschnitten vor einer operativen Entfernung dieser Gewebeabschnitte, zur Blutungsstillung oder zum Zusammenheften operativ getrennter Gewebeabschnitte zum Einsatz. Bei gastroenterologischen oder proktologischen Eingriffen kann somit beispielsweise eine Verletzung eines Darmabschnittes abgeklemmt werden oder eine Blutung des Gewebes während und nach der Intervention verringert werden. Zudem kann ein Gewebeclip auch verwendet werden, um nach der Entfernung eines Anteils der Wand des Verdauungstraktes die bestehenden Wundränder zusammenzuheften, um die Wundheilung zu beschleunigen und ein Austreten des Magen- oder Darminhalts in die Bauchhöhle zu verhindern. Um einen Einschnitt beispielsweise in den Darm zuverlässig zu verschließen, muss ein Gewebeclip eine gewisse Kraft auf die beiden zusammengehefteten Gewebestücke aufbringen. Dies stellt hohe Anforderungen an die mechanischen Materialeigenschaften des Gewebeclips. Aufgrund der hervorragenden mechanischen Eigenschaften nicht resorbierbarer medizinischer Materialien wie beispielsweise Titan, Nickel-Titan Legierungen oder nicht oxidierender Federstähle, sind herkömmliche Gewebeclips daher meist aus derartigem, nicht biologisch resorbierbarem aber gewebeverträglichem Material gefertigt.

Die Fertigung eines Gewebeclips aus einem nicht biologisch resorbierbaren Material bringt jedoch den Nachteil mit sich, dass der in den Körper eingebrachte Gewebeclip nach der Ausheilung des zusammengehefteten Gewebes ggf interventionelliv entfernt werden muss. Dies bedeutet, dass zusätzlich zu dem Eingriff zum Einbringen des Gewebeclips ein zweiter Eingriff zum Entfernen des Gewebeclips notwendig sein kann. Um diese Notwendigkeit eines zweiten interventionellen Eingriffs zum Entfernen des Gewebeclips zu umgehen, kann der Gewebeclip ganz oder teilweise aus biologisch resorbierbarem Material ausgebildet werden.

### Stand der Technik

Ein vollständig biologisch resorbierbarer Gewebeclip ist beispielsweise aus der DE 29923545U1 bekannt. Dieser Gewebeclip wird insbesondere bei Leistenbruchoperationen verwendet, um ein in die Bauchhöhle eingebrachtes Kunststoffnetz an der Bauchwand zu fixieren, bis das Kunststoffnetz fest mit der Bauchwand verwachsen ist. Der Gewebeclip besteht aus einem Körper aus resorbierbarem Material mit einer Schulter, Führungs- und Drehachsen und mit je einem Greifzahn an jeweils einem Ende der Schulter, der senkrecht von der Schulter aufragt. Mittig eingelassen in die Schulter ist ein Kniehebel zum Krümmen der Schulter. Wird der Kniehebel in eine stabile gestreckte Lage gedrückt, so wird dadurch die Schulter gekrümmt und die beiden Greifzähne schließen sich in den zwischen den Greifzähnen gebildeten Einklemmschlitz für Gewebe. Die Klemmkraft, die die Greifzähne in dem Gewebe verankert, wird hierbei durch die Hebelwirkung des Kniehebels aufgebracht.

Für viele endoskopische oder chirurgische Anwendungen ist es allerdings vorteilhaft, wenn die Klemmkraft, die die Greifzähne des Gewebeclips in dem Gewebe verankert, durch eine Federwirkung selbsttätig aufgebracht wird, wie dies beispielsweise in der US 6,849,078 B2 beschrieben ist. Dies hat beispielsweise den Vorteil, dass der hieraus bekannte Gewebeclip in einer Position mit vorgespannten Federelementen und geöffnetem Einklemmschlitz beispielsweise auf einer Endoskopspitze an das zusammenzuheftende Gewebe herangeführt werden kann und sich der vorgespannte Gewebeclip bei Aufbringung auf das Gewebe aufgrund der Federwirkung der vorgespannten Federelemente automatisch in dem Gewebe verankert, wenn das Endoskop wieder zurückgezogen wird und der Gewebeclip nicht mehr durch eine Applikationsvorrichtung in der geöffneten Position mit vorgespannten Federelementen gehalten wird. Ein zusätzliches Aufbringen von Druck auf den Gewebeclip (wie beispielsweise zum Durchdrücken eines Kniehebels erforderlich) entfällt somit bei diesem Gewebeclip mit Federelementen als selbsttätigem Schließmechanismus. Dies ermöglicht eine Anwendung eines Gewebeclips mit Federelementen auch bei empfindlichen Gewebearten oder in endoskopisch oder operativ schwer zugänglichen Regionen, wo sich ein externes Aufbringen eines Klemmdrucks auf den Gewebeclip als schwierig erweist.

Das Dokument EP2449983 A offenbart einen chirurgischen Clip aus einem geschlossenen Ring mit einer Vielzahl von Gewebegreifabschnitten, von denen jeder mit wenigstens einem Zahn ausgebildet ist, und aus einer Vielzahl von elastisch verformbaren Scharnierabschnitten, die in Umfangsrichtung zwischen zwei benachbarten Greifabschnitten angeordnet sind.

Aufgrund der oft unzureichenden mechanischen Eigenschaften von biologisch resorbierbarem Material ist es jedoch sehr schwierig, einen Gewebeclip mit selbsttätigem Schließmechanismus vorzugsweise in Form von Federelementen vollständig resorbierbar auszubilden, da zumindest die Federelemente aus einem anderen, nicht biologisch resorbierbaren gewebeverträglichen Material mit besseren mechanischen Eigenschaften (z. B. Federstahl oder eine Formgedächtnislegierung) gefertigt werden müssen.

Um die notwendigen mechanischen Eigenschaften für etwaige Federelemente des Gewebeclips aufzubringen, aber dennoch die Vorteile eines resorbierbaren Gewebeclips zu erreichen, können Gewebeclips grundsätzlich auch nur teilweise aus biologisch resorbierbarem Material ausgebildet werden. Bei diesen teilresorbierbaren Gewebeclips besteht ein Anteil des Gewebeclips aus einem biologisch resorbierbaren Material und ein anderer Anteil besteht aus einem gewebeverträglichen nicht resorbierbaren Material.

Die Klemmkraft eines solchen teilresorbierbaren Gewebeclips, mittels welcher dieser zwei Gewebeabschnitte zusammenhält und selber an dem Gewebe gehalten wird, nimmt mit zunehmender Resorption des aus biologisch resorbierbaren Material gefertigten Anteils des Gewebeclips ab, bis sich der Gewebeclip selbsttätig von dem Gewebe ablöst und der verbleibende Anteil aus nicht biologisch resorbierbarem Material beispielsweise bei Anwendung im Verdauungstrakt ohne weitere Intervention ausgeschieden werden kann.

Bei teilresorbierbaren Gewebeclips besteht jedoch das grundsätzliche Problem, dass, nach Resorption des biologisch resorbierbaren Anteils des Gewebeclips, der aus einem nicht resorbierbaren Material gefertigte Anteil des Gewebeclips in mehrere kleinere Einzelteile zerfällt (fragmentiert), welche jeweils zu klein sein können, um vom Körper regulär ausgeschieden zu werden und daher beispielsweise im Verdauungstrakt verbleiben können oder durch ihre scharfkantige Beschaffenheit den Darm schädigen können.

### Kurzbeschreibung der Erfindung

Ausgehend von dem vorstehend beschriebenen Stand der Technik liegt der Erfindung die Aufgabe zugrunde, einen teilresorbierbaren Gewebeclip zu schaffen, welcher die notwendige Klemmkraft für ein zuverlässiges Zusammenheften beispielsweise zweier Bereiche der Darmwand selbsttätig aufbringt und auch bei empfindlichem Gewebe verwendbar ist, dabei jedoch aufgrund seiner biologisch resorbierbaren Anteile die mögliche Notwendigkeit eines zweiten interventionellen Eingriffs zum Entfernen des Gewebeclips umgeht und zudem unproblematisch ausscheidbar ist.

Diese Aufgabe wird gelöst durch einen Gewebeclip mit den Merkmalen gemäß dem Anspruch 1. Vorteilhafte Weiterbildungen der Erfindung sind Gegenstand der Unteransprüche.

Der Kerngedanke der vorliegenden Erfindung besteht demzufolge darin, den Gewebeclip in Bereichen außerhalb seines selbsttätig wirkenden Schließmechanismus Federelemente) aus einem resorbierbaren Material zu fertigen. Diese Bereiche sind so ausgewählt, dass der Gewebeclip nach Auflösung des resorbierbaren Materials nach wie vor in einem Stück erhalten bleibt und trotzdem die Beiß-/Haltewirkung am Patientengewebe zumindest soweit reduziert/aufgehoben wird, dass sich der Gewebeclip vorzugsweise eigenständig vom Patientengewebe leicht ablöst.

Konstruktiv umsetzbar ist der erfindungsgemäße Kerngedanke, indem die für den unmittelbaren Klemm-/Beißeingriff oder -kontakt mit dem Patientengewebe vorgesehenen Bereiche des Gewebeclip und/oder die die Klemm-/Beißkraft übertragenden Bereiche (welche vorzugsweise den Kontaktbereichen unmittelbar nachgeordnet sind und daher die Kontaktbereiche stützen/versteifen) aus dem resorbierbaren Material zumindest anteilsmäßig bestehen, sodass nach dessen Resorption die Beißwirkung des Gewebeclip aufgehoben oder geschwächt ist. Der Gewebeclip bleibt dabei zumindest in seiner Grundstruktur/Gerüst erhalten und zerfällt nicht in Einzelteile/Fragmente.

Ein solcher teilresorbierbarer Gewebeclip zum Zusammenheften von Gewebe weist konkreter eine Anzahl von Greifzähnen auf, die das Gewebe greifen und auf einer Anzahl an sich gegenüberliegenden Kraftableitungselementen (Zahnleisten/Kiefer) angebracht sind. Zwischen den Greifzähnen wird somit ein Einklemmschlitz zum Einklemmen von Gewebe gebildet. Die Kraftableitungselemente sind im wesentlichen vorzugsweise langgestreckte, weiter vorzugsweise gewölbte Platten/Plättchen, deren Längsachse rechtwinklig zu der jeweiligen Längsachse des jeweils daran ausgebildeten Greifzahns verläuft und die dazu dienen, die durch das Einklemmen von Gewebe zwischen den Greifzähnen auf die Greifzähne aufgebrachte Klemm-/Beißkraft als Schubkraft aufzunehmen und in den Gewebeclip abzuleiten. Zum Aufbringen der für das zuverlässige Zusammenheften von Gewebesektionen erforderten Beißkraft sind die Kraftableitungselemente mit (vorzugsweise einstückig oder auch mehrteilig daran ausgebildeten) Federelementen verbunden, welche die sich gegenüberliegenden Kraftableitelemente gegeneinander vorspannen.

Gemäß einem bevorzugten Aspekt der Erfindung sind nunmehr Greifzähne des Gewebeclips aus biologisch resorbierbarem Material ausgebildet.

Mit zunehmender Resorption der Greifzähne verringert sich die Kontaktfläche zwischen den Greifzähnen und dem zwischen den Greifzähnen eingeklemmten Gewebe, folglich die Klemmkraft, die den Gewebeclip an dem Gewebe hält. Fällt die Größe der Kontaktfläche bzw. der Klemmkraft unter einen bestimmten Wert, so löst sich der verbleibende Anteil des Gewebeclips (d.h. der aus nicht resorbierbarem Material gefertigte Anteil mit gegebenenfalls anhaftendem teilweise noch unresorbiertem Anteil aus resorbierbarem Material) von dem Gewebe und kann als Ganzes beispielsweise bei einer Anwendung im Verdauungstrakt von dem Körper regulär ausgeschieden werden.

Gemäß einem zweiten Aspekt der Erfindung ist ein Anteil mindestens eines Kraftableitungselements zur gezielten Materialschwächung dieses Kraftableitungselements aus biologisch resorbierbarem Material ausgebildet.

Mit zunehmender Resorption des biologisch resorbierbaren Anteils des wenigstens einen Kraftableitungselements geht eine gezielte Materialschwächung des Kraftableitungselements einher. Dadurch kann die auf die Greifzähne wirkende Klemm-/Beißkraft nicht mehr von den Greifzähnen in den restlichen Körper des Gewebeclips und in die Federelemente abgeleitet werden, d. h. die Kraftableitelemente können der Klemm-/Beißkraft nicht mehr widerstehen und deformieren sich. Dadurch verringert sich die von den Federelementen eingeleitete Klemm-/Beißkraft an den Greifzähnen. Somit löst sich der Gewebeclip mit zunehmender Resorption des aus biologisch resorbierbarem Material gefertigten Anteils des mindestens einen Kraftableitungselements leicht aus dem Patientengewebe und kann ganzheitlich ausgeschieden werden.

Gemäß einem weiteren Aspekt der Erfindung ist der Gewebeclip in der Form eines Maulteils (Haifischmaul) ausgebildet, wonach die Kraftableitungselemente einen Ober- und Unterkiefer bilden, die an ihren Längsseiten mittels der Federelemente elastisch für ein selbsttätiges Zuklappen/Zubeißen miteinander verbunden sind. Die Federelemente können so ausgeformt sein, dass sie sowohl die Aufgabe des Schließmechanismus (Vorspannelemente) als auch gleichzeitig der Schwenkscharniere übernehmen. D. h. die Ober- und Unterkiefer sind ausschließlich über die Federelemente beidseits miteinander gekoppelt. Alternativ hierzu besteht aber auch die Möglichkeit, die Kiefer über Schwenkscharniere (Scharnierbolzen und Ösen) zu koppeln und die Vorspannkraft durch separate Federelemente parallel zu den Schwenkscharnieren aufzubringen.

Neben dem aus einem biologisch resorbierbaren Material gefertigten Anteil (Greifzähne bzw. Anteil eines Kraftableitungselements) weist der Gewebeclip auch einen aus einem nicht biologisch resorbierbaren, gewebeverträglichen Material gefertigten Anteil auf. Dieser aus einem nicht biologisch resorbierbaren, gewebeverträglichen Material gefertigte Anteil dient dazu, die notwendigen mechanischen Eigenschaften wie beispielsweise eine hohe Federelastizität und Biegefestigkeit des Gewebeclips zu gewährleisten.

In einer Ausführungsform ist der aus einem nicht biologisch resorbierbaren, gewebeverträglichen Material gefertigte Anteil des Gewebeclips einstückig, vorzugsweise im Spritzgussverfahren (Metallspritzguss), gefertigt oder aus einem Federstahlblech geschnitten insbesondere gestanzt / gelasert. Die vorzugsweise einstückige Fertigung des nicht biologisch resorbierbaren Anteils des Gewebeclips vereinfacht das Herstellungsverfahren des Gewebeclips und verbessert mechanische Eigenschaften wie beispielsweise die Biegefestigkeit des Gewebeclips. Dabei sei an dieser Stelle aber ausdrücklich darauf hingewiesen, dass der Clip (d. h. dessen Metallkörper) auch in einer mehrteiligen Konstruktion, beispielsweise mit zwei aneinander scharnierten (Bolzen-Ösen-Design) Kiefern ausführbar ist, mit welcher andere Vorteile wie z.B. die Verbesserung der Federcharakteristik, etc. erzielbar wären.

Da der Gewebeclip in sich stabil bzw. steif sein muss, muss der aus biologisch resorbierbarem Material gefertigte Anteil fest mit dem aus nicht biologisch resorbierbarem, jedoch gewebeverträglichen Material gefertigten Anteil verbunden sein. In einer Ausführungsform ist daher der aus biologisch resorbierbarem Material gefertigte Anteil form- und/oder reibschlüssig mit dem aus nicht biologisch resorbierbarem gewebeverträglichem Material gefertigten Anteil verbunden. Er kann aber auch geklebt oder angeschweißt sein. Beispielsweise können Greifzähne an den Kraftableitungselementen angezapft/angenietet oder aufgesteckt sein. Die Materialschwächungen im Bereich der Kraftableitungselemente könnten dadurch erreicht werden, indem die Kraftableitungselemente eine Art von Festeröffnungen aufweisen, die durch das resorbierbare Material ausgefüllt sind. Löst sich dieses Material auf, bleiben die Kraftableitungselemente quasi wie Fensterrahmen übrig und können keine Schubkräfte mehr aufnehmen.

In einer Ausführungsform ist das nicht biologisch resorbierbare, gewebeverträgliche Material Nitinol. Das biologisch resorbierbare Material kann ein Copolymerisat aus Milch- und Glycolsäure (PLGA), Hydroxyapatit (HPA), Beta-Tricalciumphosphat oder Tricalciumphosphat (TCP) sein. Auch wären Materialien wie Polylactid (Polymilchsäuren), PLGA (ist eine Art Derivat aus denen), etc. denkbar.

Durch Auswahl eines spezifischen biologisch resorbierbaren Materials oder durch Einstellung der Zusammensetzung des biologisch resorbierbaren Materials kann die Resorptionsrate des biologisch resorbierbaren Materials und somit der Zeitpunkt, zu dem sich der Gewebeclip selbsttätig von dem Gewebe ablöst, eingestellt werden.

Zudem kann das biologisch resorbierbare Material medizinische Wirkstoffe oder Medikamente enthalten, die mit der Zeit durch Resorption des biologisch resorbierbaren Materials oder Diffusion aus diesem biologisch resorbierbaren Material lokal freigesetzt werden. So kann der Gewebeclip auch im Rahmen der in-situ Applikation von Medikamenten Verwendung finden.

Die Form des aus biologisch resorbierbarem Material gefertigten Anteils des Gewebeclips kann den Erfordernissen einer jeweiligen Anwendung angepasst werden. Beispielsweise können die aus biologisch resorbierbarem Material gefertigten Greifzähne in ihrer Länge, Breite oder Krümmung variiert werden. Zusätzlich zu den Greifzähnen kann der aus biologisch resorbierbarem Material gefertigte Anteil des Gewebeclips auch eine biologisch resorbierbare Platte aufweisen, auf/an der die Greifzähne angebracht/ausgebildet sind und die im Wesentlichen parallel zu dem mindestens einen Kraftableitungselement verläuft. Zum Fixieren der Zähne-Platte aus biologisch resorbierbaren Material an dem nicht biologisch resorbierbaren Anteil der Kraftableitungselemente kann zudem die Platte eine Vertiefung aufweisen, in die das Kraftableitungselement des nicht biologisch resorbierbaren Anteils eingelassen ist. Zudem kann die Platte mindestens eine Erhebung aufweisen, die in eine Aussparung des nicht biologisch resorbierbaren Anteils eingreift.

Auch bei einer gezielten Materialschwächung des mindestens einen Kraftableitungselements durch Ausbildung eines Anteils des Kraftableitungselements aus biologisch resorbierbarem Material kann die Form des biologisch resorbierbaren Anteils auf die jeweiligen Erfordernisse einer spezifischen Anwendung zugeschnitten werden. Hierbei kann der biologisch resorbierbare Anteil eines Gewebeclips auch aus einer beliebigen Kombination verschieden geformter Elemente bestehen.

In einer Ausführungsform ist der aus biologisch resorbierbarem Material gefertigte Anteil des mindestens einen Kraftableitungselements im Wesentlichen als flache (rechteckige Platte ausgebildet, deren Längsachse parallel zu dem durch die Greifzähne gebildeten Einklemmschlitz für Gewebe verläuft. Der aus nicht biologisch resorbierbarem Material gefertigte Anteil des mindestens einen Kraftableitungselements weist eine entsprechende Aussparung (Fensteröffnung) auf, in die der biologisch resorbierbare Anteil des mindestens einen Kraftableitungselements vorzugsweise form- oder reibschlüssig eingelassen/eingesetzt ist.

In einer anderen Ausführungsform ist der aus biologisch resorbierbarem Material gefertigte Anteil des mindestens einen Kraftableitungselements als eine Anzahl von in Serie angeordneten flachen Platten ausgebildet, deren Längsachsen jeweils parallel zu dem durch die Greifzähne gebildeten Einklemmschlitz für Gewebe verlaufen. Der aus nicht biologisch resorbierbarem Material gefertigte Anteil des mindestens einen Kraftableitungselements weist eine Anzahl an entsprechenden Aussparungen auf, wobei jeweils eine flache Platte des biologisch resorbierbaren Anteils des mindestens einen Kraftableitungselements vorzugsweise form- oder reibschlüssig in jeweils eine Aussparung des nicht biologisch resorbierbaren Anteils eingelassen ist.

Alternativ oder zusätzlich zu den flachen Platten des biologisch resorbierbaren Anteils entsprechenden Aussparungen weist der aus nicht biologisch resorbierbarem Material gefertigte Anteil eine Aussparung in Form eines Langloches oder mehrere Aussparungen in Form mehrerer in Serie angeordneter Langlöcher auf, wobei die Längsachse eines jeden Langlochs im Wesentlichen parallel zu dem durch die Greifzähne gebildeten Einklemmschlitz für Gewebe verläuft. Ein Langloch bezeichnet hierbei eine Aussparung, deren schmale Seiten jeweils durch Halbkreise abgeschlossen sind, deren Durchmesser der Breite des Langlochs entsprechen, wobei die Längsseiten des Langloches parallel zueinander verlaufen. Auch wäre eine Kreis- oder Ellipsenform denkbar. Der biologisch resorbierbare Anteil ist hierbei als ein oder mehrere flache Platten ausgebildet, wobei eine Platte einen resorbierbaren Anteil bezeichnet, bei dem jede der Schmalseiten der Platte durch jeweils einen Halbkreis abgeschlossen ist. Eine jede Platte des biologisch resorbierbaren Anteils entspricht somit jeweils einer Aussparung in Form eines Langlochs des nicht biologisch resorbierbaren Anteils.

In einer weiteren Ausführungsform ist der aus biologisch resorbierbarem Material gefertigte Anteil des mindestens einen Kraftableitungselements als eine Anzahl an Platten ausgebildet, wobei jede Platte in jeweils eine entsprechende Aussparung in dem nicht biologisch resorbierbaren Anteil eingelassen ist. Hierbei entspricht die Anzahl an Platten der Anzahl an Greifzähnen und die Längsachse einer jeden Platte verläuft im Wesentlichen rechtwinklig zu dem durch die Greifzähne gebildeten Einklemmschlitz für Gewebe. Die Platten können hierbei so angeordnet sein, dass jeweils eine Platte mit einem der Greifzähne fluchtet.

In einer weiteren Ausführungsform ist der aus biologisch resorbierbarem Material gefertigte Anteil des mindestens einen Kraftableitungselements so ausgebildet, dass er auf seiner den Greifzähnen zugewandten Seite die Zick-Zack-Form der Greifzähne nachbildet oder auch parallel zu dem Einklemmschlitz ausgebildet ist, während er auf seiner der Außenkante des Kraftableitungselements zugewandten Seite die Form der Außenkante des Kraftableitungselements nachbildet.

Zur verbesserten Fixierung des aus biologisch resorbierbarem Material gefertigten Anteils des mindestens einen Kraftableitungselements an dem aus nicht resorbierbarem Material gefertigten Anteil kann der biologisch resorbierbare Anteil durch mindestens eine aus einem nicht biologisch resorbierbaren, gewebeverträglichen Material gefertigte Klammer in Position gehalten werden.

In einer weiteren Ausführungsform ist der aus biologisch resorbierbarem Material gefertigte Anteil des mindestens einen Kraftableitungselements als zwei von den beiden Außenseiten auf den aus nicht resorbierbarem Material gefertigten Anteil aufgebrachten und miteinander fest verbundenen Platten aus biologisch resorbierbarem Material ausgebildet. Hierbei sind die beiden Platten des aus biologisch resorbierbarem Material gefertigten Anteils des mindestens einen Kraftableitungselements über mindestens einen aus biologisch resorbierbarem Material gefertigten Stift mittels einem medizinischem Kleber, beispielsweise Cyanoacrylat, miteinander verbunden und an dem nicht biologisch resorbierbaren Anteil gehalten.

### Figurenbeschreibung

Weitere Vorteile und Merkmale der vorliegenden Erfindung ergeben sich aus der folgenden beispielhaften Beschreibung einer besonders bevorzugten Ausführungsform der Erfindung anhand der Figuren.

### Dabei zeigt

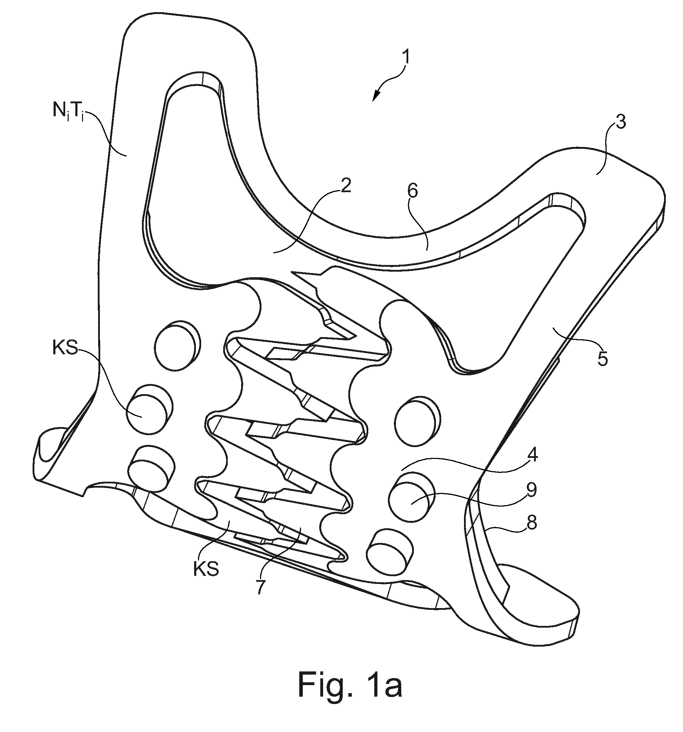

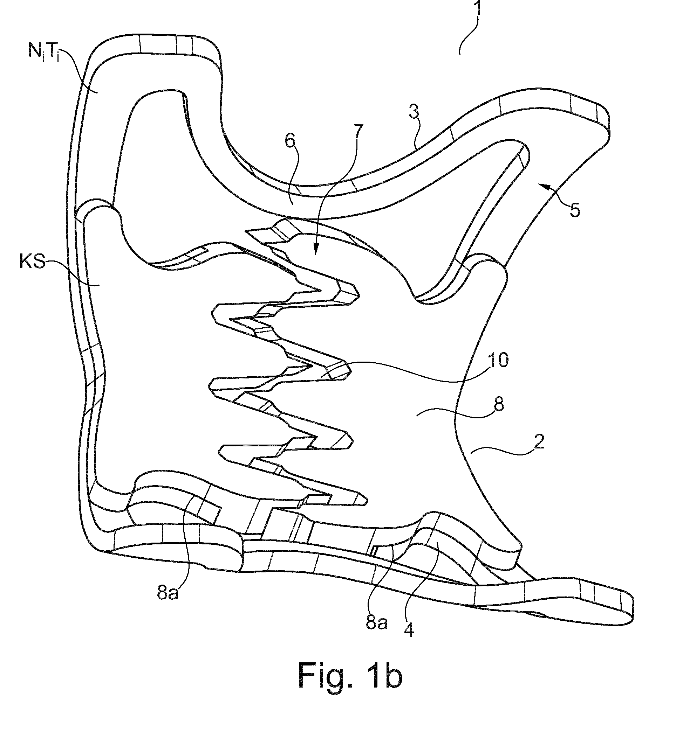

Fig. 1a eine Perspektivenansicht eines teilresorbierbaren Gewebeclip mit Greifzähnen aus biologisch resorbierbarem Material und Fig. 1b eine Ansicht der Unterseite des Gewebeclips aus Fig. 1a,

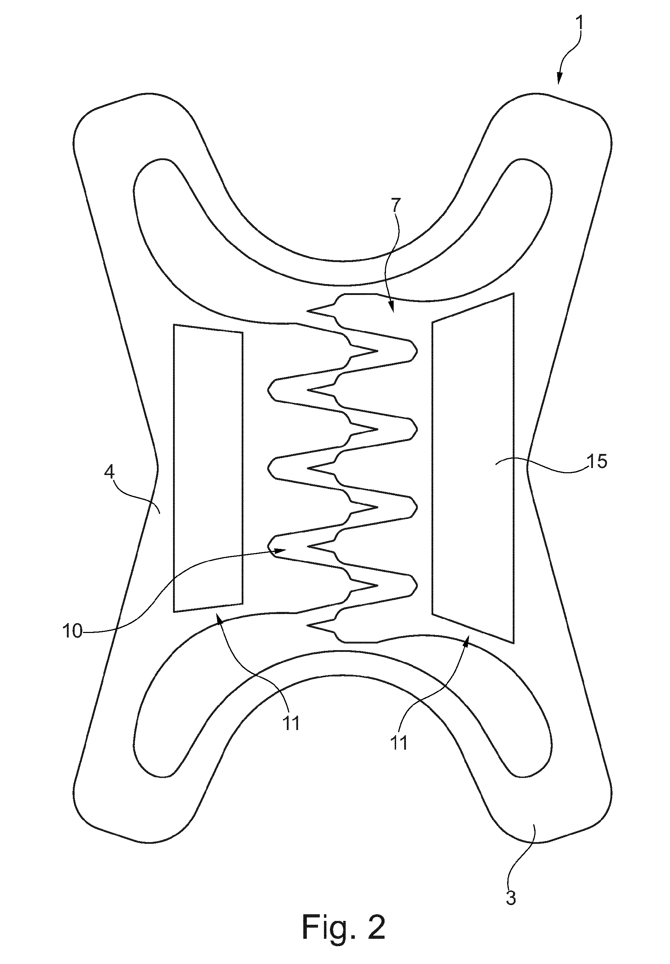

Fig. 2 einen teilresorbierbaren Gewebeclip, bei dem ein Anteil eines jeden Kraftableitungselements als ein flacher Quader aus biologisch resorbierbarem Material ausgebildet ist. Fig. 2 stellt dabei die Ansicht als Stanzteil oder lasergeschnittenes Bauteil aus einem Flachmaterial dar. Beispielsweise kann durch Weiterbearbeitung (prägen in ein U-Profil) die Funktionsform hergestellt werden.

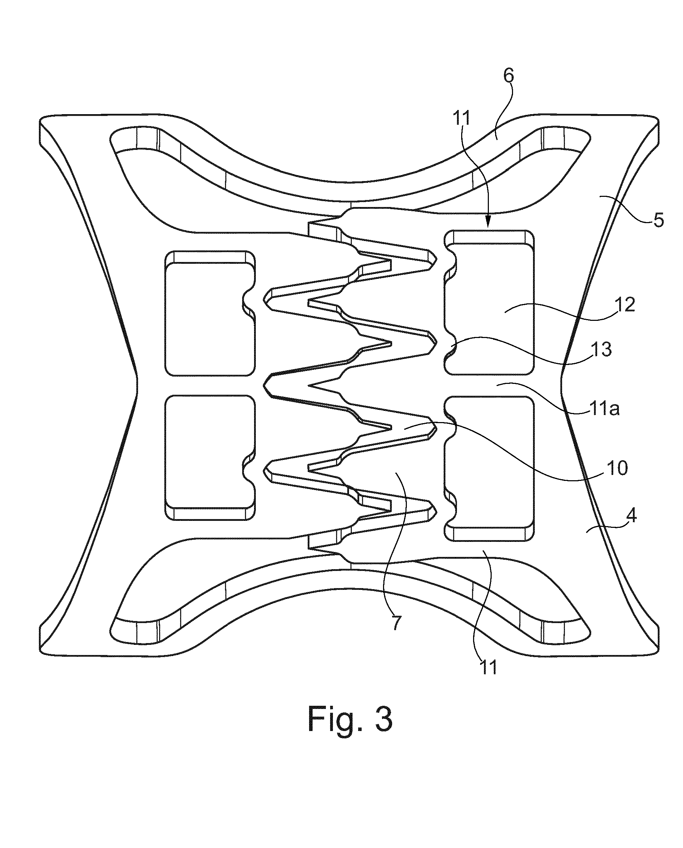

Fig. 3 einen nicht biologisch resorbierbaren Anteil eines teilresorbierbaren Gewebeclips, bei dem ein Anteil eines jeden Kraftableitungselements als zwei in Serie angeordnete flache Quader aus biologisch resorbierbarem Material ausgebildet ist.

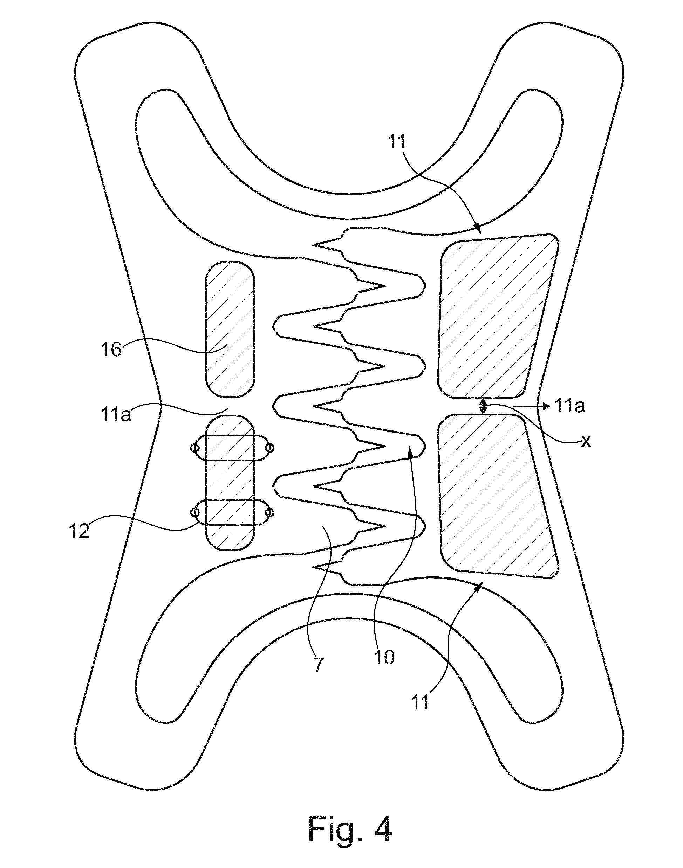

Fig. 4 einen teilresorbierbaren Gewebeclip, bei dem ein biologisch resorbierbarer Anteil eines Kraftableitungselements als zwei in Serie angeordnete Zweikreisquader ausgebildet ist und der biologisch resorbierbare Anteil eines anderen Kraftableitungselements als zwei in Serie angeordnete Elemente ausgebildet ist, die jeweils auf ihrer dem Einklemmschlitz für Gewebe zugewandten Seite parallel zu dem Einklemmschlitzverlaufen und auf ihrer der Außenkante des Kraftableitungselements zugewandten Seite parallel zu dieser Außenkante verlaufen.

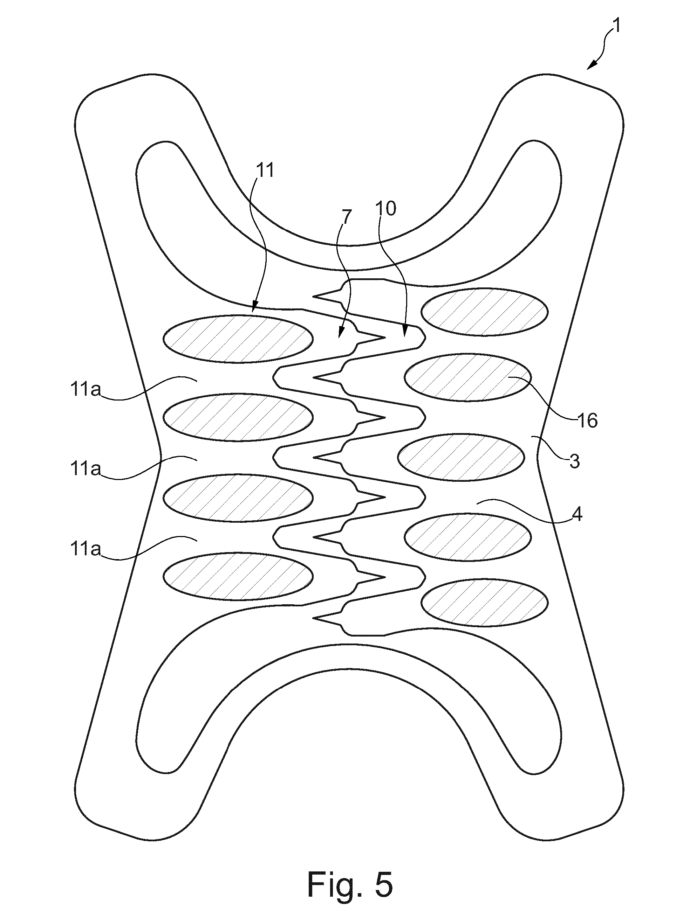

Fig. 5 einen teilresorbierbaren Gewebeclip, bei dem der biologisch resorbierbare Anteil eines jeden Kraftableitungselements als eine Anzahl an Zweikreisquadern, die jeweils mit einem Greifzahn fluchten, ausgebildet ist, wobei die Anzahl an Zweikreisquadern der Anzahl an Greifzähnen entspricht und die Längsachse eines jeden Zweikreisquaders im Wesentlichen rechtwinklig zu dem durch die Greifzähne gebildeten Einklemmschlitz für Gewebe verläuft.

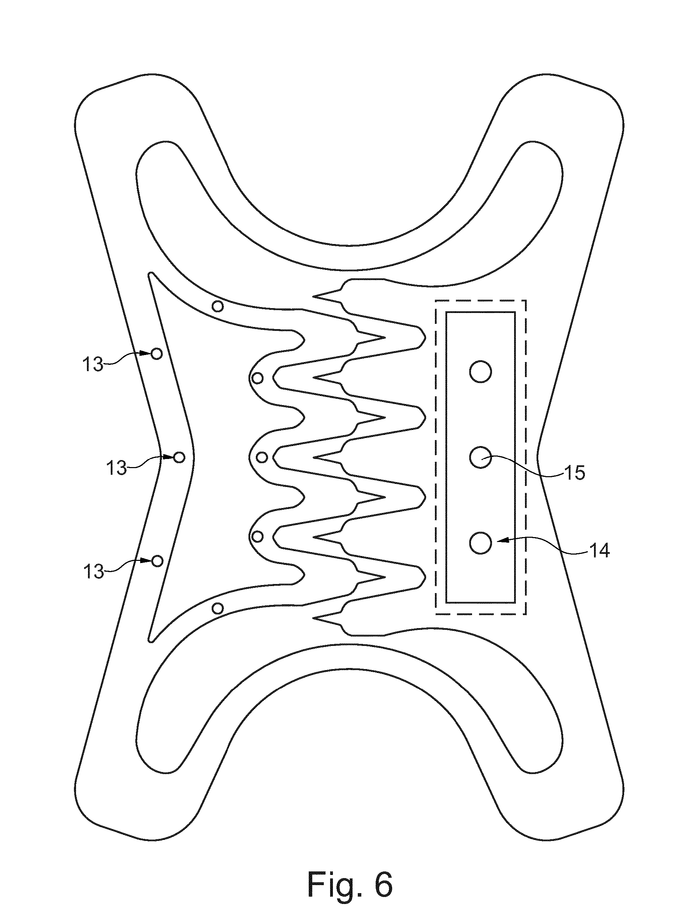

Fig.6 einen teilresorbierbaren Gewebeclip, bei dem ein biologisch resorbierbarer Anteil eines Kraftableitungselements so ausgebildet ist, dass er auf seiner den Greifzähnen zugewandten Seite die Zick-Zack-Form der Greifzähne nachbildet, während er auf seiner der Außenkante des Kraftableitungselements zugewandten Seite die Form der Außenkante des Kraftableitungselements nachbildet. Der biologisch resorbierbare Anteil eines anderen Kraftableitungselements ist als zwei von den beiden Außenseiten auf den aus nicht resorbierbarem Material gefertigten Anteil aufgebrachten und miteinander fest verbundenen Platten aus biologisch resorbierbarem Material ausgebildet, wobei die beiden Platten über drei aus biologisch resorbierbarem Material gefertigte Stifte mittels einem medizinischem Kleber, vorzugsweise Cyanoacrylat, miteinander verbunden und an dem nicht biologisch resorbierbaren Anteil gehalten sind. Ein bevorzugter Gewebeclip dieser Gattung ist hinsichtlich seiner grundsätzlichen Konstruktion mit Ausnahme der Integration von Clipbereichen aus resorbierbarem Material allgemein bekannt. Zum besseren Verständnis wird dieser Clip nachstehend unter Verweis auf die Fig. 1a näher beschrieben, welche eine mögliche Clipkonstruktion zeigt, bei welcher die Erfindung verwirklicht werden kann.

Demzufolge besteht ein solcher Clip 1 gemäß dieser bevorzugten Variante aus einer maulartigen Klemmeinrichtung mit zwei gezahnten Kiefern (Kraftableitelemente) 4, welche über zwei seitliche Scharniere 3, 5, 6 bzw. über flexible Ausformungen auf- und zugeklappt werden können. Die Scharniere 3, 5, 6 bzw. die flexiblen Ausformungen sind dabei vorzugsweise aus federelastischen Bändern ausgebildet, welche beim Aufklappen der Kiefer 4 eine Federenergie speichern, die beim Freigeben der Kiefer 4 d.h. bei einem Auslösen der Scharniere 3, 5, 6 bzw. der flexiblen Ausformungen zu einem Zuschnappen der Kiefer 4 mit vorbestimmter Klemmkraft führt.

Im Einzelnen ist jeder Clip 1 einstückig aus einem Federblech gestanzt oder gelasert, indem aus dem Federblech ein Ring mit partiell unterschiedlicher Ringbreite herausgearbeitet wird. Zwei diametrisch gegenüberliegende Ringabschnitte mit großer Ringbreite bilden die beiden Kiefer 4 (Kraftableitelemente), wohingegen die beiden dazwischen liegenden Ringabschnitte mit schmaler Ringbreite die Scharniere 3, 5, 6 bzw. die flexiblen (elastischen) Ausformungen (Biegefedern) ergeben. Insbesondere besteht vorliegend jedes Scharnier aus einem C-förmigen Federabschnitt 6 der über Abwinklungen 3 in einen Verbindungabschnitt 5 übergeht, der wiederum am zugehörigen Kiefer 4 endet. Die Kiefer 4 sind dadurch ausgebildet, indem die Ringabschnitte mit großer Ringbreite über deren Flachseite zusätzlich bogenförmig gewölbt werden, um im aufgeklappten Zustand einen Kreis zu bilden. Durch diese besondere Formgebung des gelaserten Federblechs entsteht die Form einer Art Haifischmaul mit zwei aufeinander sich zu bewegenden Zahnreihen, welche durch Lasern der Ringabschnitte mit großer Ringbreite gebildet werden.

Die Funktionsweise des vorstehend beschriebenen medizinischen Gewebeclip 1 lässt sich wie Folgt beschreiben:
Im Allgemeinen stellt eine endoskopische Implantation einer medizinischen Vorrichtung insgesamt ein den Patienten nur gering belastendes Verfahren dar. In diesem Fall muss die medizinische Vorrichtung von der Innenseite eines Hohlorgans an diesem fixiert werden. Zu diesem Zweck werden eine Anzahl (mindestens eine) der vorstehend beschriebenen Gewebeklammern, Clips oder Anker mittels eines Endoskops oder eines anderen, vorzugsweise schaftartigen Zuführmittels in das Hohlorgan eingeführt und an vorbestimmten Stellen an der Organinnenseite platziert. Hiefür wird der jeweilige Clip oder Anker an das Organgewebe herangeführt und die Vorspannfeder für ein Zuschnappen des Clips oder Aufspannen des Ankers ausgelöst. Dieser hält oder klemmt daraufhin eine Gewebefalte zwischen seinen Kiefern oder seinen Haken oder Nadeln mit einer vorbestimmten Klemm oder Spreizkraft ein, wobei sich die Zähne, Haken, Nadeln oder Zacken jedes Kiefers in das Gewebe bohren und dieses vorzugsweise durchdringen.

An dieser Stelle sei darauf hingewiesen, dass der Gewebeclip, bei welchem die Erfindung zur Anwendung kommt, nicht notwendiger Weise die vorstehend beschriebene Grundform haben muss, sondern auch anderweitig ausgebildet sein kann, etwa in Form einer Ringklammer mit einer Anzahl von in Umfangsrichtung voneinander beabstandeter, radial noch innen gerichteter Zähne, welche an Federelementen ausgeformt sind, die zu einem geschlossenen Ring zusammengefasst sind. Jedoch wird im Nachfolgenden die Erfindung anhand des maulförmigen Gewebeclip beschrieben.

Figur 1a zeigt eine Perspektivenansicht des teilresorbierbaren Gewebeclips 1, welcher erfindungsgemäß einen aus einem biologisch resorbierbaren Material gefertigten Anteil 2 und einen aus einem nicht biologisch resorbierbaren Material gefertigten Anteil 3 aufweist. In dieser Ausführungsform ist der nicht biologisch resorbierbare Anteil 3 einstückig vorzugsweise aus Nitinol gefertigt und der biologisch resorbierbare Anteil ist aus einem Copolymerisat aus Milch- und Glycolsäure (PLGA) gefertigt. Der biologisch nicht resorbierbare Anteil 3 betrifft die zwei Kraftableitungselemente/Zahnleisten/Kiefer 4 und die zwei Federelemente 6, wobei die zwei Kraftableitungselemente 4 parallel zueinander angeordnet sind und jeweils an ihren beiden Längsenden über jeweils das Verbindungselement 5 mit jeweils einem Ende eines der beiden Federelemente 6 verbunden sind. Auf seiner dem anderen Kraftableitungselement 4 zugewandten Seite weist jedes der beiden Kraftableitungselemente 4 eine Mehrzahl an Greifzähnen 7 auf. Die Greifzähne 7 sind in diesem Beispiel aus dem biologisch resorbierbaren Material gefertigt.

Der aus biologisch resorbierbarem Material gefertigte Anteil 2 des Gewebeclips 1 besteht vorliegend aus zwei Elementen, die jeweils eine Reihe von Greifzähnen 7, sowie jeweils eine Platte 8 aus biologisch resorbierbarem Material aufweisen, an der die jeweiligen Greifzähne ausgeformt sind. Jedes der Elemente des biologisch resorbierbaren Anteils 2 ist einstückig vorzugsweise aus PLGA gefertigt. Eine jede Platte 8 ist parallel zu jeweils einem Kraftableitungselement 4 angeordnet und weist eine Vertiefung auf, in die das Kraftableitungselement 4 eingelassen ist. Die einstückig mit der Platte 8 gefertigten Greifzähne 7 stehen hierbei von dem jeweiligen Kraftableitungselement 4 hervor, so dass zwischen den beiden parallel zueinander angeordneten Kraftableitungselementen 4 ein Einklemmschlitz für Gewebe gebildet wird, in den die Greifzähne 7 hineinragen. Zur festen Verbindung einer jeweiligen Platte 8 mit jeweils einem Kraftableitungselement 4 weist die Platte 8 an ihrem den Greifzähnen 7 abgewandten Ende eine Vertiefung/Materialabtrag 8a (siehe Fig. 1 b) auf, in die das Kraftableitungselement 4 eingesetzt ist. Zur Fixierung des Kraftableitungselements 4 in der Vertiefung weist die Platte 8 auf der Vertiefungsfläche eine Anzahl von Zapfen/Stiften 9 auf, die in entsprechende Aussparungen in dem jeweiligen Kraftableitungselement 4 eingreifen und die Platte 8 formschlüssig an dem Kraftableitungselement 4 halten.

Fig. 1b zeigt die Unter-/Rückseite des in Fig. 1a gezeigten Gewebeclips 1. Hier ist deutlich zu erkennen, dass der Gewebeclip 1 aus einem einstückigen Anteil (Cliprahmen) 3 aus nicht resorbierbarem Material und einem Anteil 2 aus biologisch resorbierbarem Material, welcher aus zwei Elementen besteht, zusammengesetzt ist, die an dem nicht resorbierbaren Clipanteil 3 fixiert sind. Jedes Element des biologisch resorbierbaren Anteils ist einstückig gefertigt und weist die Platte 8 und die Mehrzahl an Greifzähnen 7 auf, die über das jeweilige Kraftableitungselement 4 hervorstehen und den Einklemmschlitz 10 für Gewebe bilden. In dieser Darstellung ist erkennbar, dass die Platte 8 an ihrem an dem Kraftableitungselement 4 anliegenden Abschnitt flächige Vertiefung(Materialabtrag aufweist, in die das Kraftableitungselement 4 eingelassen ist. Die Greifzähne 7 und die Platte 8 sind vollständig aus biologisch resorbierbarem Material gefertigt. Die Vertiefung in der Platte 8 ist so beschaffen, dass sich bei in die Vertiefung eingelassenem Kraftableitungselement 4 ein kontinuierliches, glattes Oberflächenprofil zwischen dem eingelassenen Kraftableitungselement 4 und einem jeden Greifzahn 7 ergibt.

Wird ein solcher Gewebeclip praktisch eingesetzt, dringen die Zähne 7 infolge der Federkraft der beiden Federelemente 3, 5, 6 in ein Patientengewebe und verankern sich dort. Dabei wird die Beißkraft über die Platte 8 und die Kraftableitelemente 4 als Schubkräfte überragen, wobei die Kraftableitelemente 4 gleichzeitig die Platte 8 stabilisieren. Löst sich das absorbierbare Material auf, bleibt am Ende des Auflösungsprozesses nur noch der nicht absorbierbare Clipanteil ganzheitlich übrig, der eine nur geringe Klemmkraft auf das Gewebe noch ausüben kann. Der Clip kann sich daher leicht vom Gewebe lösen.

Für eine verbesserte Greifwirkung der Greifzähne 7 kann es vorteilhaft sein, die Greifzähne 7 aus einem nicht biologisch resorbierbaren Material wie Nitinol auszubilden und statt dessen zur gezielten Materialschwächung mindestens eines der Kraftableitungselemente einen Anteil/Bereich dieses Kraftableitungselements aus biologisch resorbierbarem Material auszubilden.

Ein solcher Gewebeclip 1 mit Greifzähnen 7 aus nicht resorbierbarem Material ist in Fig. 2 dargestellt. Der Anteil 2 aus biologisch resorbierbarem Material ist hier als zwei Elemente ausgebildet, die jeweils in Form eines flachen Quaders (rechteckige Platte) 15 ausgebildet sind und jeweils in einem der Kraftableitungselemente 4 eingelassen/eingesetzt sind. Ein jedes der Kraftableitungselemente 4 weist eine der Form eines Elements des biologisch resorbierbaren Anteils 2 entsprechende Aussparung/Fensteröffnung auf, in die jeweils ein Element des biologisch resorbierbaren Anteils 2 eingelassen ist. Die Aussparung in dem Kraftableitungselement 4 kann beispielsweise durch selektives Ätzen oder Senkerodieren hergestellt (hierbei würde sich bevorzugt um einen gesamten Bereich ein ggf. noch immer geschlossenes Profil ausbilden, welches aber im Vergleich zur sonstigen Clip-Materialstärke sehr dünn sein könnte und dadurch erheblich flexibler verformbar wäre. Der resorbierbare Anteil würde dann die dünne Materialstärke auffüllen/ausgleichen.) oder einfach aus den Kraftableitelementen 4 ausgeschnitten sein (hierbei entsteht dann ein offenes fensterförmiges Durchgangsloch). Die Längsachse eines jeden in ein Kraftableitungselements 4 eingelassenen Elements des biologisch resorbierbaren Anteils 2 verläuft bevorzugt parallel zu dem durch die Greifzähne 7 gebildeten Einklemmschlitz 10 für Gewebe. Die beiden Längskanten eines jeden Elements des biologisch resorbierbaren Anteils 2 verlaufen im Wesentlichen parallel zu einander. Hierbei ist ein jedes Element des biologisch resorbierbaren Anteils 2 so ausgebildet, dass zwischen einer jeden kurzen Kante eines jeden Elements und der nächstliegenden kurzen Kante des Kraftableitungselements, in welches das Element eingelassen ist, nur noch jeweils ein dünner Steg 11 aus nicht biologisch resorbierbarem Material besteht (Bildung einer Rahmenstruktur im jeweiligen Kraftableitelement).

Ist der biologisch resorbierbare Anteil 2 des Gewebeclips 1 resorbiert, wird die gesamte Klemm-/Beißkraft auf die dünnen Stege zwischen den Fensteröffnungen aufgebracht, die sich dadurch elastisch verformen/ausknicken. Dadurch nimmt die durch den Gewebeclip 1 auf das Gewebe aufgebrachte Klemmkraft ab und der Gewebeclip 1 löst sich von dem Gewebe ab. Um diesen Effekt zu verstärken, können die dünnen Stege 11 aus Nitinol oder einem Material mit einem besonders hohen Nitinolanteil gefertigt werden. Die dünnen Stege 11 verhindern zudem ein Auseinanderfallen des Clip in mehrere Teile des biologisch nicht resorbierbaren Anteils 3 nach Resorption des biologisch resorbierbaren Anteils 2 des Gewebeclips 1, so dass der verbleibende Anteil 3 des Gewebeclips 1 einstückig bestehen bleibt und regulär und sicher vom Körper ausgeschieden werden kann.

Um die Stabilität des Gewebeclips 1 zu verbessern, so dass dieser nach Resorption des biologisch resorbierbaren Anteils 2 eine verbesserte Klemmkraft aufweist, kann - wie in Fig. 3 gezeigt - der aus nicht biologisch resorbierbarem Material gefertigte Anteil 3 des Gewebeclips 1 mittig in jedem der Kraftableitungselemente 4 einen zusätzlichen dünnen Steg 11 a zusätzlich zu den dünnen Stegen 11 an den jeweiligen kurzen Kanten eines jeden Kraftableitungselements 4 aufweisen. Ein jedes Element des biologisch resorbierbaren Anteils 2 des Gewebeclips 1 ist hierbei als zwei in Serie angeordnete, über den mittig in dem Kraftableitungselement angeordneten dünnen Steg 11 a getrennte flache Quader (rechteckige Platten) 15 ausgebildet. Zur Aufnahme der in Serie angeordneten Platten 15 des biologisch resorbierbaren Anteils 2 weist ein jedes der zwei Kraftableitungselemente 4 zwei in Serie angeordnete Aussparungen/Fensteröffnungen 12 auf, die der Form der biologisch resorbierbaren Platten 15 entsprechen. Die Längsachse einer jeden Aussparung 12 verläuft im Wesentlichen parallel zu der Längsachse des Einklemmschlitzes 10. Zur verbesserten Verankerung jeweils einer Platte 15 des biologisch resorbierbaren Anteils 2 in einer der Aussparungen 12 eines Kraftableitungselements 4 weist die den Greifzähnen 4 zugewandte Kante einer jeden Aussparung 12 einen oder mehrere Höcker 13 Positionsnasen auf, die in die Aussparung 12 hineinragen und jeweils mit einem Tal der durch die Greifzähne 7 definierten Zick-Zack-Form fluchten. Die Platten 15 des biologisch resorbierbaren Anteils weisen jeweils eine oder mehrere Nuten auf, in die jeweils einer der Höcker 13 eingreift und den biologisch resorbierbaren Anteil 2 somit an dem nicht biologisch resorbierbaren Anteil 3 fixiert.

Wie in Fig. 4 gezeigt, kann die exakte Form der Elemente des biologisch resorbierbaren Anteils 2 des Gewebeclips 1 auf die Erfordernisse einer spezifischen Anwendung zugeschnitten werden. Es können auch in verschiedene Kraftableitungselemente 4 verschieden geformte biologisch resorbierbare Elemente eingesetzt werden. So sind in Fig. 4 bei einem Kraftableitungselement 4 die Aussparungen zur Aufnahme der Elemente des biologisch resorbierbaren Anteils 2 als zwei in Serie angeordnete Langlöcher ausgeführt, deren jeweilige Längsachse im Wesentlichen parallel zu der Längsachse des Einklemmschlitzes 10 verläuft. Zwischen den beiden Langlöchern befindet sich ein dünner Steg 11a. Die biologisch resorbierbaren Elemente sind als jeweils einem Langloch entsprechende Platten 16 ausgebildet, die in jeweils ein Langloch eingelassen sind. Zusätzlich kann der nicht biologisch resorbierbare Anteil 3 Klammern 12 aufweisen, mit denen die Elemente des biologisch resorbierbaren Anteils 2 in den Aussparungen des nicht biologisch resorbierbaren Anteils 3 gehalten werden.

Alternativ können die Aussparungen in dem nicht biologisch resorbierbaren Anteil 3 des Gewebeclips 1 auch so ausgebildet sein, dass die den Greifzähnen 7 zugewandte Seite einer jeden Aussparung im Wesentlichen parallel zu der Längsachse des Einklemmschlitzes 10 verläuft. Die dem mittigen dünnen Steg 11 a zugewandte kurze Seite einer jeden Aussparung verläuft rechtwinklig zu der zu der Längsachse des Einklemmschlitzes 10. Die der Außenkante des Kraftableitungselements 4 zugewandte Längsseite einer jeden Aussparung, sowie die dem äußeren dünnen Steg 11 zugewandte kurze Seite einer jeden Aussparung verlaufen im Wesentlichen parallel zu der nächstliegenden Außenkante des Kraftableitungselements 4.

Fig. 5 zeigt einen Gewebeclip 1, bei dem der biologisch resorbierbare Anteil 2 aus mehreren flachen Platten 16 pro Kraftableitelement besteht, die jeweils in ein entsprechendes Langloch in einem Kraftableitungselement 4 eingelassen sind. Hierbei verläuft die Längsachse einer jeden Platte 16 im Wesentlichen rechtwinklig zu der Längsachse des Einklemmschlitzes 10. Jede Platte 16 ist so angeordnet, dass sie mit jeweils einem Greifzahn 7 fluchtet. Dünne Stege 11 a trennen jeweils eine Platte 16 von der nächstliegenden Platte 16.

In Figur 6 ist ein Gewebeclip 1 gezeigt, in dessen eines Kraftableitungselement 4 ein Element eines biologisch resorbierbaren Anteils 2 eingelassen ist, wobei dieses Element so ausgebildet ist, dass es auf seiner den Greifzähnen 7 zugewandten Seite die Zick-Zack-Form der Greifzähne 7 nachbildet, während es auf seiner der Außenkante des Kraftableitungselements 4 zugewandten Seite die Form der Außenkante des Kraftableitungselements 4 nachbildet. Zur verbesserten Fixierung des biologisch resorbierbaren Elements in dem Kraftableitungselement 4 weist die Aussparung in dem Kraftableitungselement an ihrer Innenkante Stifte (Zapfen) 13 auf, die in entsprechende Löcher in dem in die Aussparung eingelassenen Element des biologisch resorbierbaren Anteils 2 eingreifen. Das in das andere Kraftableitungselement 4 des Gewebeclips 1 eingelassene Element des biologisch resorbierbare Anteils 2 ist als zwei von den beiden Außenseiten auf den aus nicht resorbierbarem Material gefertigten Anteil 3 aufgebrachte und miteinander fest verbundene Platten 14 aus biologisch resorbierbarem Material ausgebildet, wobei die beiden Platten 14 über den Umfang der Aussparung in dem Kraftübertragungselement 4 hervorstehen (gestrichelte Linie) und über drei aus biologisch resorbierbarem Material gefertigten Stifte 15 mittels eines medizinischen Klebers, vorzugsweise Cyanoacrylat, miteinander verbunden und an dem nicht biologisch resorbierbaren Anteil 3 gehalten sind.

## Patentansprüche

1. Teilresorbierbarer Gewebeclip mit
mindestens einem Greifzahn, der das Gewebe greift,
mindestens einem Kraftableitungselement, das mit mindestens einem Greifzahn bestückt ist, sowie
mindestens einem Vorspannelement, das mit dem oder mit einem der Kraftableitungselemente verbunden ist, um über das Kraftableitungselement eine Beißkraft auf den mindestens einen Greifzahn anzulegen, **dadurch gekennzeichnet, dass**
der mindestens eine Greifzahn (7) aus biologisch resorbierbarem Material ausgebildet ist,
ein Teilbereich des mindestens einen oder mindestens eines der Kraftableitungselemente aus biologisch resorbierbarem Material ausgebildet ist,
und der mindestens eine Greifzahn und der Teilbereich des mindestens einen oder mindestens eines der Kraftableitungselemente miteinander über eine formschlüssig an dem Kraftableitungselement gehaltene Verbindungsplatte einstückig miteinander verbunden sind.

2. Teilresorbierbarer Gewebeclip nach einem der vorhergehenden Ansprüche, **dadurch gekennzeichnet, dass** der Gewebeclip mit Ausnahme der Anteile gemäß Anspruch 1 aus einem nicht biologisch resorbierbaren Material gefertigt ist.

3. Teilresorbierbarer Gewebeclip nach Anspruch 2, **dadurch gekennzeichnet, dass** das nicht biologisch resorbierbare Material Nitinol ist.

4. Teilresorbierbarer Gewebeclip nach einem der vorhergehenden Ansprüche, **dadurch gekennzeichnet, dass** der aus einem nicht biologisch resorbierbaren Material gefertigten Anteil des Gewebeclips einstückig, vorzugsweise im Spritzguss- Stanz oder Laserschneidverfahren gefertigt ist und vorzugsweise eine Rahmenkonstruktion bildet.

5. Teilresorbierbarer Gewebeclip nach einem der vorhergehenden Ansprüche, **dadurch gekennzeichnet, dass** das biologisch resorbierbare Material ein Copolymerisat aus beispielsweise Milch- und Glycolsäure (PLGA), Hydroxyapatit (HPA), Beta-Tricalciumphosphat, Tricalciumphosphat (TCP) oder Polylactid ist.

6. Teilresorbierbarer Gewebeclip einem der vorhergehenden Ansprüche, **dadurch gekennzeichnet, dass** durch die Wahl des biologisch resorbierbaren Materials oder die Variation der Zusammensetzung des biologisch resorbierbaren Materials der Zeitverlauf der Resorption und somit der Klemmkraft des Gewebeclips bestimmbar ist.

7. Teilresorbierbarer Gewebeclip nach einem der vorhergehenden Ansprüche, **dadurch gekennzeichnet, dass** das biologisch resorbierbare Material medizinische Wirkstoffe enthält, die mit der Zeit durch Resorption des Materials oder Diffusion aus diesem Material lokal freigesetzt werden.

8. Teilresorbierbarer Gewebeclip nach einem der vorhergehenden Ansprüche, **dadurch gekennzeichnet, dass** der aus biologisch resorbierbarem Material gefertigte Anteil formschlüssig, reibschlüssig und/oder durch eine Klebung mit dem aus nicht biologisch resorbierbarem Material gefertigten Anteil verbunden ist.

9. Teilresorbierbarer Gewebeclip nach einem der vorhergehenden Ansprüche, **dadurch gekennzeichnet, dass** der aus biologisch resorbierbarem Material gefertigte Anteil des mindestens einen Kraftableitungselements im Wesentlichen als flache Platte ausgebildet ist, deren Längsachse vorzugsweise parallel zu einem durch die Greifzähne gebildeten Einklemmschlitz für Gewebe verläuft.

10. Teilresorbierbarer Gewebeclip nach einem der vorhergehenden Ansprüche, **dadurch gekennzeichnet, dass** der aus biologisch resorbierbarem Material gefertigte Anteil des mindestens einen Kraftableitungselements als zwei nebeneinander angeordnete flache Platten ausgebildet ist, deren Längsachsen jeweils parallel zu einem durch die Greifzähne gebildeten Einklemmschlitz für Gewebe verlaufen.

11. Teilresorbierbarer Gewebeclip nach einem der vorhergehenden Ansprüche, **dadurch gekennzeichnet, dass** der aus biologisch resorbierbarem Material gefertigte Anteil des mindestens einen Kraftableitungselements durch mindestens eine aus einem nicht biologisch resorbierbaren Material gefertigte Klammer in Position gehalten wird.

12. Teilresorbierbarer Gewebeclip nach einem der vorhergehenden Ansprüche, **dadurch gekennzeichnet, dass** der aus biologisch resorbierbarem Material gefertigte Anteil des mindestens einen Kraftableitungselements auf seiner den Greifzähnen zugewandten Seite die Zick-Zack-Form der Greifzähne nachbildet, während er auf seiner dem äußeren Rand des Kraftableitungselements zugewandten Seite die Form der nächstliegenden Außenkante des Kraftableitungselements nachbildet.

13. Teilresorbierbarer Gewebeclip nach einem der vorhergehenden Ansprüche, **dadurch gekennzeichnet, dass** der aus biologisch resorbierbarem Material gefertigte Anteil des mindestens einen Kraftableitungselements als zwei von den beiden Außenseiten auf den aus nicht biologisch resorbierbarem Material gefertigten Anteil aufgebrachten, miteinander fest verbundenen Platten ausgebildet ist.

14. Teilresorbierbarer Gewebeclip nach Anspruch 13, **dadurch gekennzeichnet, dass** die beiden Platten des aus biologisch resorbierbaren Material gefertigten Anteils des mindestens einen Kraftableitungselements über mindestens einen aus biologisch resorbierbarem Material gefertigten Stift mittels medizinischem Kleber, vorzugsweise Cyanoacrylat, miteinander verbunden sind.

15. Teilresorbierbarer Gewebeclip nach Anspruch 1, **dadurch gekennzeichnet, dass** der mindestens eine Greifzahn (7) an einer Platte ausgeformt ist, welche an dem wenigstens einen Kraftableitungselement flächig fixiert ist.

## Claims

1. Partly resorbable tissue clip having
at least one gripping tooth which grips the tissue,
at least one force discharge element which is provided with at least one gripping tooth, and
at least one pretensioning element which is connected to the or to one of the force discharge elements in order to place via the force discharge element a biting force on the at least one gripping tooth, **characterised in that** the at least one gripping tooth (7) is constructed from biologically resorbable material,
a part-region of the at least one or at least one of the force discharge elements is constructed from biologically resorbable material,
and the at least one gripping tooth and the part-region of the at least one or at least one of the force discharge elements are connected to each other in an integral manner by means of a connection plate which is retained in a positive-locking manner on the force discharge element.

2. Partly resorbable tissue clip according to any one of the preceding claims, **characterised in that** the tissue clip with the exception of the portions according to claim 1 is produced from a non-biologically resorbable material.

3. Partly resorbable tissue clip according to claim 2, **characterised in that** the non-biologically resorbable material is nitinol.

4. Partly resorbable tissue clip according to any one of the preceding claims, **characterised in that** the portion of the tissue clip which is produced from a non-biologically resorbable material is produced in an integral manner, preferably with an injection-moulding, punching or laser cutting method and preferably forms a frame construction.

5. Partly resorbable tissue clip according to any one of the preceding claims, **characterised in that** the biologically resorbable material is a copolymer comprising, for example, poly lactic-co-glycolic acid (PLGA), hydroxyapatite (HPA), beta tricalcium phosphate, tricalcium phosphate (TCP) or polylactide.

6. Partly resorbable tissue clip according to any one of the preceding claims, **characterised in that**, as a result of the selection of the biologically resorbable material or the variation of the composition of the biologically resorbable material, the time course of the resorption and consequently the clamping force of the tissue clip can be determined.

7. Partly resorbable tissue clip according to any one of the preceding claims, **characterised in that** the biologically resorbable material contains medical active ingredients which are locally released over time by means of resorption of the material or diffusion from this material.

8. Partly resorbable tissue clip according to any one of the preceding claims, **characterised in that** the portion which is produced from biologically resorbable material is connected to the portion produced from non-biologically resorbable material in a positive-locking manner, frictionally engaging manner and/or by means of adhesive bonding.

9. Partly resorbable tissue clip according to any one of the preceding claims, **characterised in that** the portion of the at least one force discharge element which is produced from biologically resorbable material is constructed substantially as a flat plate, whose longitudinal axis preferably extends parallel with a clamping slot formed by the gripping teeth for tissue.

10. Partly resorbable tissue clip according to any one of the preceding claims, **characterised in that** the portion of the at least one force discharge element which is produced from biologically resorbable material is constructed as two flat plates which are arranged beside each other and whose longitudinal axes extend parallel with a clamping slot formed by the gripping teeth for tissue.

11. Partly resorbable tissue clip according to any one of the preceding claims, **characterised in that** the portion of the at least one force discharge element which is produced from biologically resorbable material is held in position by means of at least one clip which is produced from a non-biologically resorbable material.

12. Partly resorbable tissue clip according to any one of the preceding claims, **characterised in that** the portion of the at least one force discharge element which is produced from biologically resorbable material at the side thereof facing the gripping teeth reproduces the zig-zag shape of the gripping teeth whilst, at the side thereof facing the outer edge of the force discharge element, it reproduces the shape of the nearest outer edge of the force discharge element.

13. Partly resorbable tissue clip according to any one of the preceding claims, **characterised in that** the portion of the at least one force discharge element which is produced from biologically resorbable material is constructed as two plates which are securely connected to each other and which are, fitted from the two outer sides to the portion which is produced from non-biologically resorbable material.

14. Partly resorbable tissue clip according to claim 13, **characterised in that** the two plates of the portion of the at least one force discharge element which is produced from biologically resorbable material are connected to each other via at least one pin which is produced from biologically resorbable material by means of medical adhesive, preferably cyanoacrylate.

15. Partly resorbable tissue clip according to claim 1, **characterised in that** the at least one gripping tooth (7) is formed on a plate which is fixed to the at least one force discharge element in a planar manner.

## Revendications

1. Clip de tissu partiellement résorbable comprenant
au moins une dent de préhension, qui saisit le tissu,
au moins un élément de déviation de force, qui est équipé d'au moins une dent de préhension, ainsi
qu'au moins un élément de précontrainte, qui est relié à l'élément de déviation de force ou à un des éléments de déviation de force afin d'appliquer, par l'intermédiaire de l'élément de déviation de force, une force d'occlusion sur l'au moins une dent de préhension, **caractérisé en ce**
**que** l'au moins une dent de préhension (7) est réalisée à partir d'un matériau biologiquement résorbable,
**qu'**une zone partielle l'au moins un l'élément de déviation de force ou d'au moins un des éléments de déviation de force est réalisée à partir d'un matériau biologiquement résorbable,
et **que** l'au moins une dent de préhension et la zone partielle de l'élément de déviation de force ou d'au moins un des éléments de déviation de force sont reliées l'une à l'autre d'un seul tenant par l'intermédiaire d'une plaque de liaison maintenue par complémentarité de forme au niveau de l'élément de déviation de force.

2. Clip de tissu partiellement résorbable selon l'une quelconque des revendications précédentes, **caractérisé en ce que** le clip de tissu est confectionné, à l'exception des parties selon la revendication 1, à partir d'un matériau biologiquement non résorbable.

3. Clip de tissu partiellement résorbable selon la revendication 2, **caractérisé en ce que** le matériau biologiquement non résorbable est du Nitinol.

4. Clip de tissu partiellement résorbable selon l'une quelconque des revendications précédentes, **caractérisé en ce que** la partie, confectionnée à partir d'un matériau biologiquement non résorbable, du clip de tissu est confectionnée d'un seul tenant, de préférence lors du procédé de moulage par injection, du procédé d'estampage ou du procédé de découpe au laser, et forme de préférence une structure de cadre.

5. Clip de tissu partiellement résorbable selon l'une quelconque des revendications précédentes, **caractérisé en ce que** le matériau biologiquement résorbable est un copolymérisat composé par exemple d'acide lactique et d'acide glycolique (PLGA), d'hydroxyapatite (HPA), de béta-phosphate tricalcique, de phosphate tricalcique (TCP) ou de polylactide.

6. Clip de tissu partiellement résorbable selon l'une quelconque des revendications précédentes, **caractérisé en ce que** le temps de résorption et, ainsi, la force de serrage du clip de tissu peuvent être déterminés par le choix du matériau biologiquement résorbable ou par la variation de la composition du matériau biologiquement résorbable.

7. Clip de tissu partiellement résorbable selon l'une quelconque des revendications précédentes, **caractérisé en ce que** le matériau biologiquement résorbable contient des principes actifs médicaux, qui sont localement libérés dans le temps par résorption du matériau ou diffusion depuis ledit matériau.

8. Clip de tissu partiellement résorbable selon l'une quelconque des revendications précédentes, **caractérisé en ce que** la partie confectionnée à partir d'un matériau biologiquement résorbable est reliée, par complémentarité de forme, par friction et/ou par un collage, à la partie confectionnée à partir d'un matériau biologiquement non résorbable.

9. Clip de tissu partiellement résorbable selon l'une quelconque des revendications précédentes, **caractérisé en ce que** la partie, confectionnée à partir d'un matériau biologiquement résorbable, de l'au moins un élément de dérivation de force est réalisée sensiblement sous la forme d'une plaque plate, dont l'axe longitudinal s'étend de préférence de manière parallèle par rapport à une entaille d'insertion par serrage, formée par les dents de préhension, pour tissu.

10. Clip de tissu partiellement résorbable selon l'une quelconque des revendications précédentes, **caractérisé en ce que** la partie, confectionnée à partir d'un matériau biologiquement résorbable, de l'au moins un élément de dérivation de force est réalisée sous la forme de deux plaques plates disposées l'une à côté de l'autre, dont les axes longitudinaux s'étendent respectivement de manière parallèle par rapport à une entaille d'introduction par serrage, formée par les dents de préhension, pour tissu.

11. Clip de tissu partiellement résorbable selon l'une quelconque des revendications précédentes, **caractérisé en ce que** la partie, confectionnée à partir d'un matériau biologiquement résorbable, de l'au moins un élément de déviation de force est maintenue en position par au moins une agrafe confectionnée à partir d'un matériau biologiquement non résorbable.

12. Clip de tissu partiellement résorbable selon l'une quelconque des revendications précédentes, **caractérisé en ce que** la partie, confectionnée à partir d'un matériau biologiquement résorbable, de l'au moins un élément de déviation de force reproduit, sur son côté tourné vers les dents de préhension, la forme en zigzag des dents de préhension, tandis qu'elle reproduit, sur son côté tourné vers le bord extérieur de l'élément de déviation de forme, la forme de l'arête extérieure la plus proche de l'élément de déviation de force.

13. Clip de tissu partiellement résorbable selon l'une quelconque des revendications précédentes, **caractérisé en ce que** la partie, confectionnée à partir d'un matériau biologiquement résorbable, de l'au moins un élément de déviation de force est réalisée sous la forme de deux plaques appliquées depuis les deux côtés extérieurs sur la partie confectionnée à partir d'un matériau biologiquement non résorbable, reliées l'une à l'autre de manière fixe.

14. Clip de tissu partiellement résorbable selon la revendication 13, **caractérisé en ce que** les deux plaques de la partie, confectionnée à partir d'un matériau biologiquement résorbable, de l'au moins un élément de déviation de force sont reliées l'une à l'autre par l'intermédiaire au moins d'une tige confectionnée à partir d'un matériau biologiquement résorbable, au moyen d'une colle à usage médical, de préférence du cyanoacrylate.

15. Clip de tissu partiellement résorbable selon la revendication 1, **caractérisé en ce que** l'au moins une dent de préhension (7) est formée au niveau d'une plaque, qui est fixée à plat au niveau de l'au moins un élément de déviation de force.
